# EUROPÄISCHE PATENTSCHRIFT

(11) **EP 2 659 922 B1**
(45) Veröffentlichungstag und Bekanntmachung des Hinweises auf die Patenterteilung: **06.11.2019**
(21) Anmeldenummer: 13166541.6
(22) Anmeldetag: 03.05.2013
(51) Int. Cl.: A61M 5/00, B01L 9/00, B65B 55/02, B65D 77/04, B65D 5/50, B01L 9/06, B65B 63/08, B65B 3/00, A61J 1/14, B65D 85/00

(54) **Haltestruktur zum gleichzeitigen Halten einer Mehrzahl von medizinischen oder pharmazeutischen Behältern sowie Transport- oder Verpackungsbehälter mit Selbiger**
Support structure for simultaneously holding a plurality of medical or pharmaceutical containers and transport or packaging container with the same
Structure de support pour le support simultané d'une pluralité de récipients médicaux ou pharmaceutiques ainsi que récipient de transport ou d'emballage doté d'une telle structure

(30) Priorität: 03.05.2012 DE 102012103901; 29.11.2012 DE 102012111624
(43) Veröffentlichungstag der Anmeldung: 06.11.2013
(73) Patentinhaber: Schott AG, 55122 Mainz (DE)
(72) Erfinder: Wissner, Kai, 69493 Hirschberg (DE); Lovis, Ralph, 8400 Winterthur (CH); Pawlowski, Edgar, 55271 Stadecken-Elsheim (DE)
(74) Vertreter: 2K Patentanwälte Blasberg Kewitz & Reichel

(56) Entgegenhaltungen:
- EP-A1- 2 420 450
- WO-A1-96/29556
- WO-A1-2009/015862
- WO-A1-2011/135085
- US-A- 3 537 189
- US-B1- 6 341 490

## Beschreibung

### Gebiet der Erfindung

Die vorliegende Erfindung betrifft die gleichzeitige automatisierte Förderung und Übergabe einer Mehrzahl von Behältern zur Aufbewahrung von Substanzen für medizinische, pharmazeutische oder kosmetische Anwendungen an Prozessstationen, beispielsweise eine Abfüll- oder Bearbeitungsanlage, einen Steriltunnel, einen Gefriertrockenschrank zum Lyophilisieren einer wirkstoffhaltigen Flüssigkeit oder dergleichen, sowie die Verwendung eines flachen Trägers zu diesem Zweck.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoff oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in einem Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen.

Beispiele für solche Transport- und Verpackungsbehälter werden in US 8,118,167 B2 und US 8,100,263 B2 offenbart. Weitere vergleichbare Transport- und Verpackungsbehälter und Haltestrukturen werden in WO 2011/135085 A1, US 2011/0277419 A1, WO 2012/025549 A1, WO 2011/015896 A1, WO 2012/007056 A1 und WO 2009/015862 A1 offenbart.

Ein herkömmlicher Transport- und Verpackungsbehälter, wie dieser in ähnlicher Form auch in der EP 2 382 135 B1 (entsprechend der WO 2010/086128) offenbart wird, ist in der Fig. 1a dargestellt. Die Behälter 2' sind in einem Einsatz mit einer Mehrzahl von darin ausgebildeten zylindrischen Aufnahmen 39' aufgenommen, die von Querstegen 35' und einem Boden 40' ausgebildet werden. Die Böden 3' der Behälter 2' liegen auf dem Boden 40' des Einsatzes auf, die Befüllöffnungen 7' am oberen Ende der Behälter 2' sind der Öffnung des Transport- und Verpackungsbehälters 10' zugewandt. Der Einsatz wird mit der Mehrzahl von Behältern 2' in den Transport- und Verpackungsbehälter 10' eingesetzt und stellt ein separates Element dar.

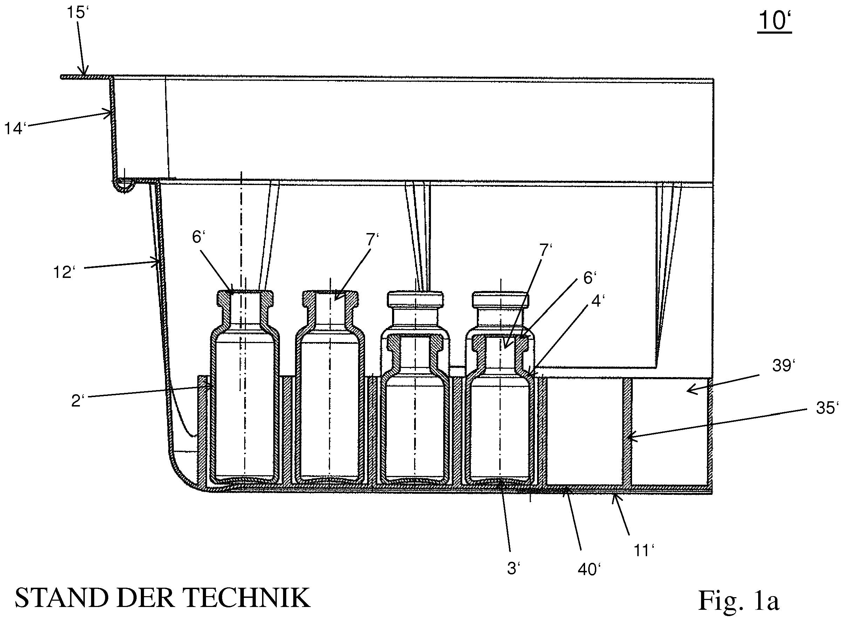

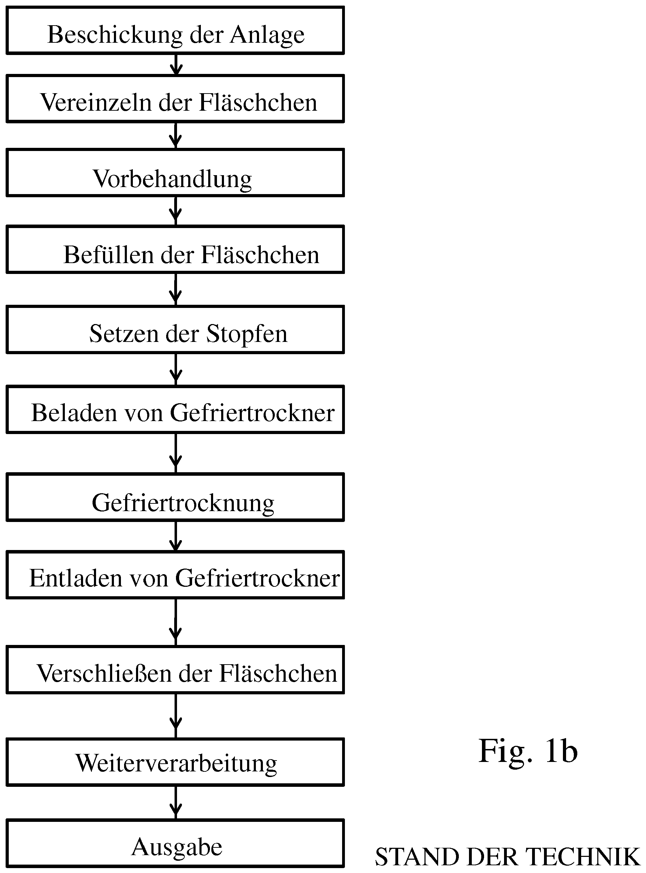

Zur Weiterverarbeitung müssen die Medikamentenbehälter jedoch stets vereinzelt werden. Dies sei beispielhaft anhand der Fig. 1b erläutert, die ein schematisches Flussdiagram eines herkömmlichen Verfahrens zur Gefriertrocknung von pharmazeutischen Präparaten in Medikamentenbehältern darstellt, wie dieses beispielsweise in der US 5,964,043 offenbart ist.

Zunächst wird die Prozessanlage, nämlich ein Steriltunnel, mit den Fläschchen beschickt. Hierzu wer-den die Fläschchen kopfüber in Transportrahmen eingehängt, die dann durch die Prozessanlage gefördert werden. Zur Vorbehandlung werden die in den Transportrahmen gehaltenen Fläschchen zunächst sterilisiert. Anschließend werden die Transportrahmen mit den darin aufbewahrten Fläschchen gewendet und mit einer Wirkstofflösung befüllt. Anschließend wird auf den oberen Rand der Fläschchen ein Stopfen aufgesetzt, in welchem ein Kanal ausgebildet ist, über den der Flascheninnenraum während der Gefriertrocknung jeweils mit der Kammer des Gefriertrockenschranks kommuniziert.

Zur Gefriertrocknung (auch als Lyophilisation oder Sublimationstrocknung bezeichnet) werden die Fläschchen dann aus dem Transportrahmen entnommen und einzeln in den Gefriertrockenschrank eingebracht. Dabei müssen die Böden der Fläschchen zur Erzielung eines guten Kühleffekts unmittelbar auf einem planar ausgebildeten Kühlboden abgesetzt werden. Wird an dieser Stelle kein vollflächiger unmittelbarer Kontakt gewährleistet, so führt dies zu einer erheblichen Verlängerung des Gefriertrocknungsprozesses, was zu höheren Kosten führt.

Nach der Gefriertrocknung werden die Fläschchen aus dem Gefriertrockenschrank entnommen, die Stopfen heruntergedrückt und ein Metalldeckel auf den Stopfen aufgesetzt und ge-bördelt oder gecrimpt. Derart verarbeitete Fläschchen werden dann ausgeliefert, beispielsweise indem eine Mehrzahl von Fläschchen gemeinsam von einem Träger aufgenommen werden und der Träger in einen Transport- und Verpackungsbehälter eingesetzt wird, der zur Auslieferung dann steril verpackt wird.

Der für die Gefriertrocknung notwendige unmittelbare Kontakt zwischen dem Boden der Medikamentenbehälter und dem Kühlboden erfordert herkömmlich eine Behandlung oder Verarbeitung einzelner Behälter, was die Verarbeitungs- und Verpackungskosten erhöht. Eine chargenweise, gleichzeitige Weiterverarbeitung einer Mehrzahl von Medikamentenbehältern ist herkömmlich nicht möglich. Jedenfalls ist ein unmittelbarer Kontakt der Böden der Medikamentenbehälter, insbesondere der Böden von Fläschchen, bei den herkömmlichen Haltestrukturen nicht möglich.

Insbesondere aufgrund der notwendigen Vereinzelung ist die vorstehend beschriebene Vorgehensweise zeitaufwändig und teuer.

EP 2 420 450 A1 offenbart eine Vorrichtung zum Befüllen von Behältern und aseptischen Bedingungen mit einem oberen Raum und einem unteren Raum, wobei in dem oberen Raum die eigentliche Behandlung der Behälter unter aseptischen Bedingungen stattfindet und sich in dem unteren Raum die Maschinerie zum Betreiben der Vorrichtung befindet. Beide Räume sind voneinander getrennt. Hierzu werden die Behälter einzeln auf einem flachen Schlitten transportiert. Ein kasten- oder wannenförmig ausgebildeter Transport- und Verpackungsbehälter, in dessen Innenraum sämtliche Behälter nach der Behandlung aufgenommen sind, ist nicht offenbart.

WO 96/29556 A1 offenbart ein Verfahren zur Gefriertrocknung von Vials, bei dem die Behälter den Prozess durchlaufen, während diese i Magazinen gehalten werden. Zwar haben diese Magazine einen flachen Boden, jedoch durchragen die Vials die Öffnungen der Magazine vollständig. Die Böden der Vials sind nicht unmittelbar auf einer Grundplatte eines Trägers abgestützt.

### Zusammenfassung der Erfindung

Aufgabe der vorliegenden Erfindung ist es, ein Verfahren zur Behandlung oder Verarbeitung von Behältern, die zur Aufbewahrung von Substanzen für kosmetische, medizinische oder pharmazeutische Anwendungen dienen oder diese enthalten, dahingehend weiterzubilden, dass dieses noch rascher und wirtschaftlicher, besser automatisierbar und zuverlässiger ausgeführt werden kann. Ein weiterer Gesichtspunkt der vorliegenden Erfindung betrifft ferner die Verwendung eines flachen Trägers zu diesem Zweck.

Diese Aufgaben werden gemäß der vorliegenden Erfindung durch ein Verfahren mit den Merkmalen nach Anspruch 1 und durch die Verwendung eines flachen Trägers nach Anspruch 15 gelöst. Weitere vorteilhafte Ausführungsformen sind Gegenstand der rückbezogenen Unteransprüche.

Gemäß der vorliegenden Erfindung wird ein Verfahren zur Behandlung oder Verarbeitung von Behältern bereitgestellt, die zur Aufbewahrung von Substanzen für kosmetische, medizinische oder pharmazeutische Anwendungen dienen oder diese enthalten, insbesondere von Fläschchen (vials), wobei die Behälter an einem Ende offen ausgebildet sind, die Behälter mittels einer Fördereinrichtung zur Behandlung oder Verarbeitung automatisiert an einer Prozessstation vorbeigeführt werden oder diese durchlaufen, und die Behälter anschließend in einen Transport- oder Verpackungsbehälter eingebracht werden, der einen Boden, eine umlaufend ausgebildete Seitenwand und eine dem Boden gegenüber liegende Einführöffnung aufweist, bei welchem Verfahren: eine Mehrzahl von Behältern auf einem flachen Träger in einer vorbestimmten Anordnung angeordnet werden, sodass die Böden der Behälter unmittelbar auf dem flachen Träger aufliegen; die Behälter, während diese auf dem flachen Träger aufliegen, von der Fördereinrichtung zu einer Prozessstation gefördert werden, um dort behandelt oder verarbeitet zu werden; und der Transport- oder Verpackungsbehälter nach der Behandlung oder Verarbeitung der Behälter so angeordnet wird, dass sämtliche Behälter der Mehrzahl von Behältern in dem Transport- oder Verpackungsbehälter aufgenommen sind. Anschließend wird der Transport- oder Verpackungsbehälter verschlossen oder versiegelt. Insbesondere wird der Transport- oder Verpackungsbehälter direkt auf den flachen Träger aufgesetzt, um diesen unmittelbar zu verschließen oder zu versiegeln. Bei dem Verfahren ist der Transport- oder Verpackungsbehälter kästen- oder wannenförmig ausgebildet, mit einem Innenraum, in dem sämtliche Behälter nach deren Behandlung oder Verarbeitung aufgenommen sind. Der Träger weist eine flache Grundplatte auf, sodass die Böden der Behälter bei dem Verfahren unmittelbar auf der Grundplatte des flachen Trägers aufliegen.

Weil die Böden der Behälter unmittelbar auf dem flachen Träger aufliegen, können die Behälter aufrecht stehend behandelt oder verarbeitet werden. In dieser Orientierung können die Behälter anschließend auch in den Transport- oder Verpackungsbehälter eingebracht werden, ohne dass diese gewendet werden müssen. Diese überraschend einfache Maßnahme minimiert das Risiko, dass die aufgrund von Erschütterungen oder Kollisionen miteinander beschädigt werden oder deren Anordnung unbeabsichtigt verändert wird, etwa durch Erschütterungen oder dergleichen. Weil die Böden der Behälter während der Behandlung oder Verarbeitung unmittelbar auf dem Boden des flachen Trägers aufliegen können, kann ferner erfindungsgemäß ein sehr guter thermischer Kontakt zu einer Auflagefläche für den flachen Träger, insbesondere einer Kühlfläche, beispielsweise eines Gefriertrocknungsschranks, gewährleistet werden. Zu diesem Zweck wird der flache Träger bevorzugt aus einem Material mit hoher Wärmeleitfähigkeit und mit einer möglichst geringen Materialstärke ausgebildet und die Rückseite des flachen Trägers, die beim bestimmungsgemäßen Gebrauch der Auflagefläche zugewandt ist, möglichst plan oder exakt korrespondierend zur Oberfläche dieser Auflagefläche ausgebildet, um einen vollflächigen Kontakt zwischen Auflagefläche und flachem Träger zu gewährleisten.

Zur Aufnahme der Behälter in dem Transport- oder Verpackungsbehälter wird dieser nach der Behandlung oder Verarbeitung einfach über den flachen Träger gestülpt oder auf diesem aufgelegt, ohne dass der flache Träger hierzu bewegt oder gewendet werden muss.

Gemäß einer weiteren Ausführungsform sind auf dem Boden des flachen Trägers oder des Transport- oder Verpackungsbehälters Positionierungsmittel ausgebildet, die mit den Behältern so zusammenwirken, um die vorbestimmte Anordnung der Mehrzahl von Behältern festzulegen. Die Positionierungsmittel auf dem flachen Träger können die vorbestimmte Anordnung auch während der Bearbeitung oder Verarbeitung der Behälter aufrechterhalten. Die Positionierungsmittel können insbesondere in Form von Vorsprüngen mit geeignetem Profil vorgesehen sein, die bevorzugt senkrecht von der Oberfläche des flachen Trägers bzw. dem Boden des Transport- oder Verpackungsbehälters vorstehen und als Anschläge bzw. Begrenzungen wirken, um eine seitliche Verschiebung der Behälter geeignet einzuschränken bzw. zu begrenzen. Hierzu kann die Höhe dieser Vorsprünge grundsätzlich auch deutlich geringer sein als die axiale Länge der Behälter. Insbesondere können diese Vorsprünge auch jeweils umlaufend ausgebildet sein, um eine in Draufsicht zylindrische oder polyedrische Aufnahme auszubilden, in welcher der Behälter abschnittsweise aufgenommen werden kann. Die Seitenwände dieser Aufnahmen können unmittelbar an den Seitenwänden der Behälter anliegen oder diese gar geringfügig klemmen. Grundsätzlich können die Positionierungsmittel bzw. Seitenwände jedoch auch so ausgelegt sein, dass die Behälter von diesen lose aufgenommen sind. Auf diese Weise kann insbesondere eine Kollision von unmittelbar benachbarten Behältern verhindert werden, während diese auf dem flachen Träger aufliegen oder in dem Transport- oder Verpackungsbehälter aufgenommen sind.

Gemäß einer weiteren Ausführungsform sind zwischen den Positionierungsmitteln Öffnungen ausgebildet, die von einer Hebeeinrichtung durchgriffen werden, um die Behälter zur Behandlung oder Verarbeitung in oder an der Prozessstation in eine angehobene Position zu heben, in welcher die Böden der Behälter nicht unmittelbar auf dem flachen Träger aufliegen. Somit können auch weitere Prozessschritte ausgeführt werden, bei denen eine solche angehobene Position erforderlich ist, beispielsweise zur Befüllung, optischen Inspektion des Inhalts der Behälter oder dergleichen. Die Hebeeinrichtung kann somit auf die Unterseite der Behälter einwirken, um diese anzuheben und wieder abzusenken, sodass das Eindringen von mechanischem Abrieb in das Innenvolumen der Behälter zuverlässig ausgeschlossen werden kann. Die Hebeeinrichtung kann beispielsweise als vertikal verstellbarer Hebearm oder - Stange ausgebildet sein, die die Öffnungen des flachen Trägers durchgreift und zum Anheben eines Behälters in Anlage mit dem Boden des Behälters gebracht wird.

Gemäß einer weiteren Ausführungsform wirken die Behälter in der angehobenen Position auch weiterhin mit den Positionierungsmitteln zusammen, sodass die vorbestimmte Anordnung in der angehobenen Position weiterhin von den Positionierungsmitteln festgelegt wird. Dies erleichtert die automatisierte Handhabung und Prozessierung der Behälter während und nach der Behandlung oder Verarbeitung der Behälter, verhindert aber gleichzeitig auch eine Kollision von unmittelbar benachbarten Behältern.

Gemäß einer weiteren Ausführungsform werden die Behälter so in dem Transport- oder Verpackungsbehälter aufgenommen, dass das offene Ende dem Boden des Transport- oder Verpackungsbehälters zugewandt ist. Wenn der Transport- oder Verpackungsbehälter verschlossen ist, sind die Behälter somit aufrecht stehend in dem Transport- oder Verpackungsbehälter aufgenommen. Die vorgenannten Positionierungsmittel können während des Transports weiterhin zur Stabilisierung der Positionen der Behälter dienen.

Während der Behandlung oder Verarbeitung sind die Behälter bevorzugt aufrecht stehend auf dem flachen Träger angeordnet. Der Transport- oder Verpackungsbehälter wird deshalb nach der Behandlung oder Verarbeitung der Behälter bevorzugt von oben her auf den Träger aufgelegt oder über diesen gestülpt. Nach dem Verschließen des Transport- oder Verpackungsbehälters kann dieser dann gewendet werden, sodass die Behälter dann kopfüber in dem Transport- oder Verpackungsbehälter angeordnet sind. Die vorgenannten Positionierungsmittel auf dem Boden des Transport- oder Verpackungsbehälters können hierzu als Aufnahmen zum teilweisen Aufnehmen oder Festlegen der Positionen der Behälter in dem Transport- oder Verpackungsbehälter ausgebildet sein.

Gemäß einer weiteren Ausführungsform ist in dem flachen Träger oder dem Transport- oder Verpackungsbehälter zumindest eine Öffnung ausgebildet, durch welche ein Gas zum Sterilisieren des Innenraums des Transport- oder Verpackungsbehälters einströmt. Diese jeweilige Öffnung kann nach dem Sterilisieren dann geeignet gasdicht und/oder steril verschlossen werden. Gemäß einer weiteren Ausführungsform ist diese jeweilige Öffnung mit einer gasdurchlässigen Kunststofffolie abgedeckt, durch die das zum Sterilisieren verwendete Gas in den Innenraum des Transport- oder Verpackungsbehälters einströmen kann.

Gemäß einer weiteren Ausführungsform wird der Transport- oder Verpackungsbehälter mittels einer gasdurchlässigen Kunststofffolie verschlossen oder versiegelt, insbesondere mittels einer Kunststofffolie, die aus einem gasdurchlässigen Geflecht von Kunststofffasern ausgebildet ist und insbesondere eine Tyveck®-Folie ist. Diese gasdurchlässige Kunststofffolie kann auch als der vorgenannte flache Träger verwendet werden. Um ein Verrutschen der Behälter auf der Kunststofffolie während der Behandlung oder Verarbeitung der Behälter zu vermeiden, können auf der Unterseite der Behälter oder auf der Oberfläche der Kunststofffolie geeignete reibungsmindernde Beschichtungen vorgesehen sein oder können Haft- oder Klebepunkte vorgesehen sein, die zweckmäßig aus einem nicht ausgasenden Haft- oder Klebemittel ausgebildet sind.

Gemäß einer weiteren Ausführungsform werden jeweils benachbarte Träger zur Behandlung oder Verarbeitung so miteinander unmittelbar verbunden, dass diese relativ zueinander in einer Längsrichtung und/oder in einer Querrichtung des Trägers unverschieblich sind und die unmittelbar benachbarten Träger gemeinsam an der Prozessstation vorbeigeführt werden oder diese durchlaufen. Die Träger können somit zu größeren Einheiten verkettet werden, was deren Handhabung und Förderung in einer Prozessanlage vereinfachen kann.

Hierzu können an Rändern von unmittelbar benachbarten Trägern zueinander korrespondierend ausgebildete Formschlussgebilde ausgebildet, die miteinander zur Behandlung oder Verarbeitung der Behälter in einen formschlüssigen Eingriff überführt werden. Die Grundflächen der Aussparungen und/oder Vorsprünge können, jeweils in Draufsicht betrachtet, insbesondere verschieden zu einer Rechteckform sein und unmittelbar korrespondierend zueinander ausgebildet sein, sodass ein Formschluss in der Art einer einfachen Schwalbenschwanzverbindung realisiert werden kann, die eine besondere stabile Verbindung von benachbarten Trägern ermöglicht, die einfach wieder gelöst werden kann.

Gemäß einer weiteren Ausführungsform sind die Formschlussgebilde an einem ersten der beiden benachbarten Träger eine elastische Zunge mit einem daran darauf ausgebildeten Rastvorsprung oder einer darauf ausgebildeten Rastaussparung sowie an dem zweiten der beiden benachbarten Träger eine korrespondierend zu dem Rastvorsprung ausgebildete Aufnahme oder einen korrespondierend zu der Rastaussparung ausgebildeten Vorsprung aufweisen, die zur Verbindung der benachbarten Träger miteinander in den vorgenannten formschlüssigen Eingriff überfuhrt werden.

Zur Reduzierung der Grundfläche eines flachen Trägers können dessen Ränder als abnehmbare oder wegschwenkbare Elemente ausgebildet sein und zur Behandlung oder Verarbeitung abgenommen oder weggeschwenkt werden. Dies kann den Durchsatz von Prozessanlagen weiter erhöhen.

Ein weiterer Gesichtspunkt der vorliegenden Erfindung betrifft die Verwendung eines flachen Trägers, wie in der vorliegenden Anmeldung offenbart, der zum Verschliessen oder Versiegeln eines kasten- oder wannenförmig ausgebildeten Transport- oder Verpackungsbehälters dient, der einen Boden, eine umlaufend ausgebildete Seitenwand zum Ausbilden eines Innenraums des Transport- oder Verpackungsbehälters und eine dem Boden gegenüber liegende Einführöffnung aufweist, oder der in dem verschlossenen oder versiegelten Transport- oder Verpackungsbehälter zum Transport aufgenommen ist, als Träger für eine Mehrzahl von Behältern, die zur Aufbewahrung von Substanzen für kosmetische, medizinische oder pharmazeutische Anwendungen dienen oder diese enthalten, insbesondere von Fläschchen (vials), bei deren Behandlung oder Verarbeitung an oder in einer Prozessstation, wobei die Mehrzahl von Behältern in einer vorbestimmten Anordnung so an dem flachen Träger angeordnet sind, sodass die Böden der Behälter unmittelbar auf dem flachen Träger aufliegen. Dieser Träger ist erfindungsgemäß so ausgebildet, wie in der vorliegenden Anmeldung offenbart, wobei die Mehrzahl von Behältern in einer vorbestimmten Anordnung so an dem flachen Träger angeordnet sind, dass die Böden der Behälter unmittelbar auf der Grundplatte des flachen Trägers aufliegen, wobei sämtliche Behälter der Mehrzahl von Behältern in dem Innenraum des kasten- oder wannenförmigen Transport- oder Verpackungsbehälters aufgenommen sind.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a: einen Transport- und Verpackungsbehälter gemäß dem Stand der Technik;
- Fig. 1b: ein Flussdiagramm eines herkömmlichen Verfahrens zur Behandlung oder Verarbeitung von Behältern;
- Fig. 2a - 2b: in einer perspektivischen Explosionsansicht sowie in einem Teilschnitt einen Transport- und Verpackungsbehälter sowie einen flachen Träger gemäß einer ersten Ausführungsform der vorliegenden Erfindung;
- Fig. 2c - 2d: in einer perspektivischen Explosionsansicht sowie in einem Teilschnitt einen Transport- und Verpackungsbehälter sowie einen flachen Träger gemäß einer zweiten Ausführungsform der vorliegenden Erfindung;
- Fig. 2e: den Transport- und Verpackungsbehälter gemäß der Fig. 2d in einer schematischen Draufsicht auf den Boden desselben;
- Fig. 3a - 3b: in einer perspektivischen Explosionsansicht sowie in einem Teilschnitt einen Transport- und Verpackungsbehälter sowie einen flachen Träger gemäß einer dritten Ausführungsform der vorliegenden Erfindung;
- Fig. 4a - 4b: in einer perspektivischen Draufsicht sowie in einer Draufsicht einen Transport- und Verpackungsbehälter zur Verwendung bei einem Verfahren gemäß der vorliegenden Erfindung;
- Fig. 4c: in einer perspektivischen Draufsicht sowie in einem Teilschnitt einen Transport- und Verpackungsbehälter zur Verwendung bei einem weiteren Verfahren gemäß der vorliegenden Erfindung;
- Fig. 5: in einer perspektivischen Draufsicht sowie in einem Teilschnitt einen Transport- und Verpackungsbehälter gemäß einer weiteren Ausführungsform der vorliegenden Erfindung;
- Fig. 6a: ein Flussdiagramm eines Verfahrens zur Behandlung oder Verarbeitung von Behältern gemäß der vorliegenden Erfindung;
- Fig. 6b: in einer schematischen Draufsicht die Anwendung eines Verfahrens gemäß der vorliegenden Erfindung zur Gefriertrocknung einer Substanz in den Behältern;
- Fig. 7a: einen Prozessschritt des erfindungsgemäßen Verfahrens, bei dem ein Behälter mittels einer Hubeinrichtung, die eine Öffnung eines flachen Trägers oder des Transport- und Verpackungsbehälters durchgreift, zur Behandlung oder Verarbeitung in eine angehobene Position überführt wird;
- Fig. 7b: eine Variante eines flachen Trägers oder einer Haltestruktur gemäß der vorliegenden Erfindung, mit Vorsprüngen und Aussparungen an den abnehmbaren oder wegschwenkbaren Elementen, die einer weiteren Erhöhung der Packungsdichte dienen;
- Fig. 7c: in einer schematischen Draufsicht eine weitere Variante der Vorsprünge oder Aussparungen gemäß der Fig. 7b;
- Fig. 7d: in einer stark vergrößerten Teildraufsicht die Verbindung zweier flacher Träger oder Haltestrukturen gemäß einer weiteren Ausführungsform; und
- Fig. 7e: einen Querschnitt entlang A-A gemäß der Fig. 7d.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

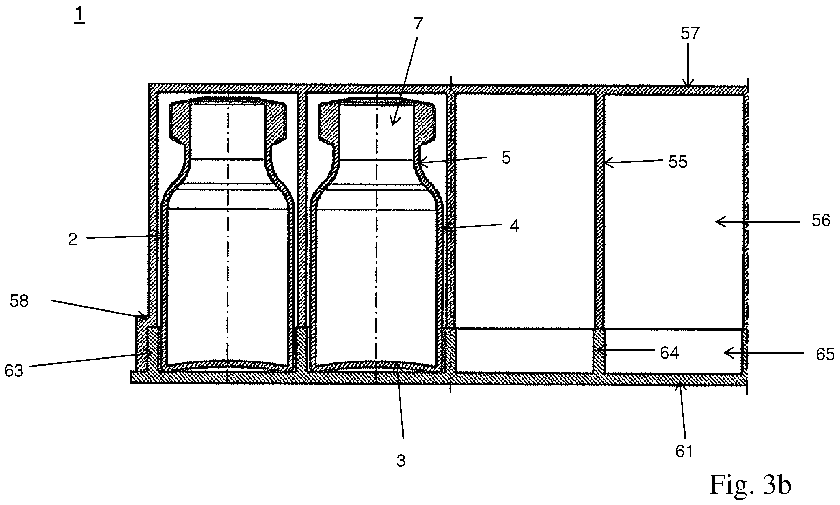

Ein Beispiel für Medikamentenbehälter im Sinne der vorliegenden Anmeldung in Gestalt von Fläschchen ist in der Fig. 3b schematisch in einem Längsschnitt dargestellt. Diese weisen eine zylindrische Grundform auf, mit einer zylinderförmigen Seitenwand 4 mit - im Rahmen der Toleranzen - konstantem Innen- und Außendurchmesser, die von einem flach ausgebildeten Flaschenboden 3 senkrecht abragt und nahe dem oberen offenen Ende 7 des Fläschchens 2 in einen verengten Halsabschnitt 5 von vergleichsweise geringer axialer Länge und anschließend in einen verbreiterten oberen Rand 6 (auch Rollrand) übergeht, der einen größeren Außendurchmesser aufweist als der zugeordnete Halsabschnitt 5 und zur Verbindung mit einem Verschlusselement ausgelegt ist. Wie man der Fig. 3b entnehmen kann, ist die Unterseite des Rollrands 6 abgeschrägt ausgebildet und erstreckt sich unter einem spitzen Winkel abwärts und hin zu dem verengten Halsabschnitt 5. Wenngleich in der Fig. 3b dargestellt ist, dass der Boden 3 leicht einwärts gewölbt ausgebildet ist, kann es bevorzugt sein, wenn der Boden 3 flach ausgebildet ist, insbesondere um einen möglichst vollflächigen Kontakt zu einer Auflage- oder Kühlfläche, auf welcher das Fläschchen aufliegt, zu gewährleisten.

Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauern viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil. Unweigerlich weisen solche Behälter herstellungsbedingt Toleranzen auf, die gerade bei Glasbehältern einen oder mehrere Zehntel Millimeter betragen können.

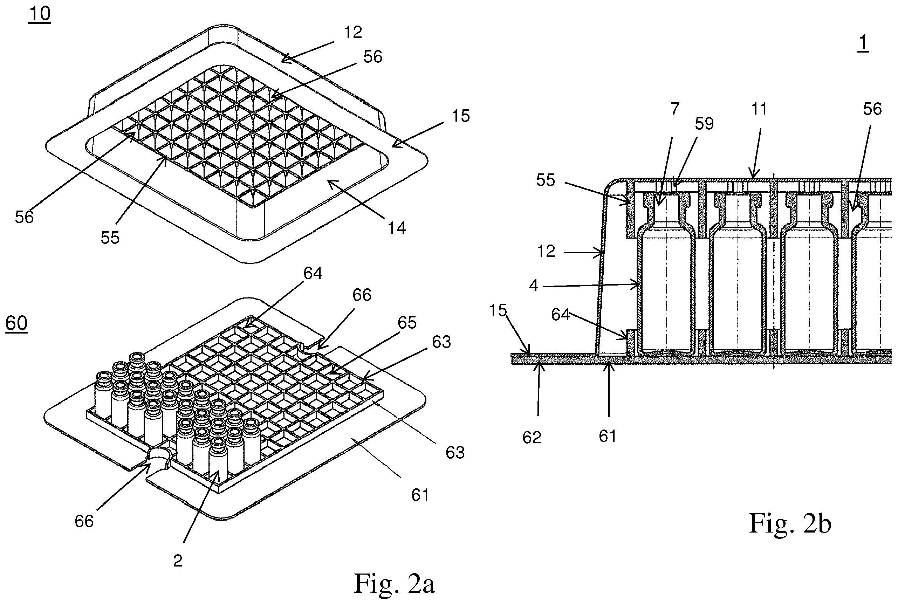

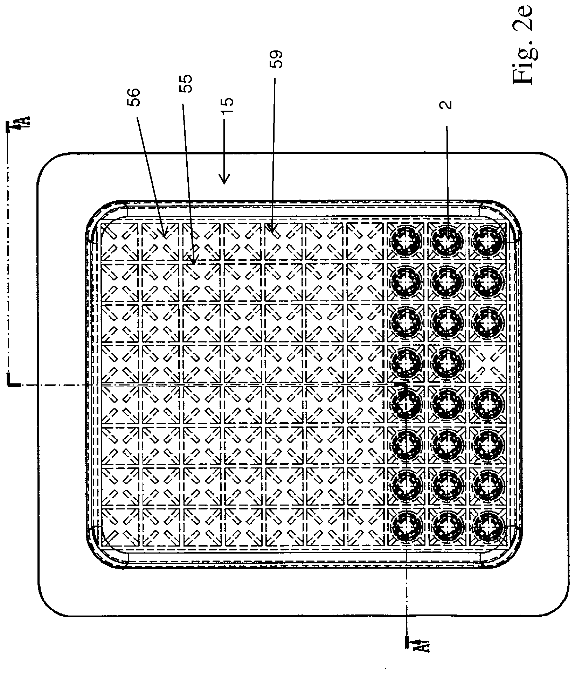

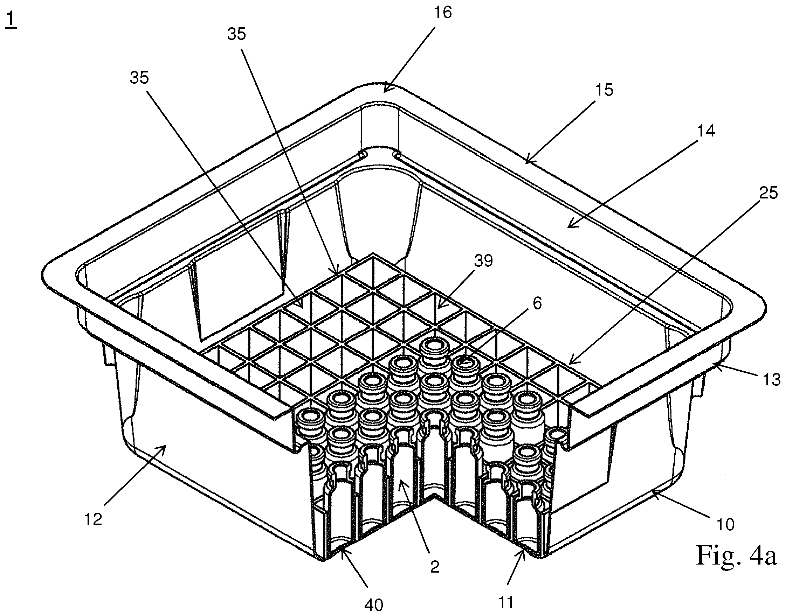

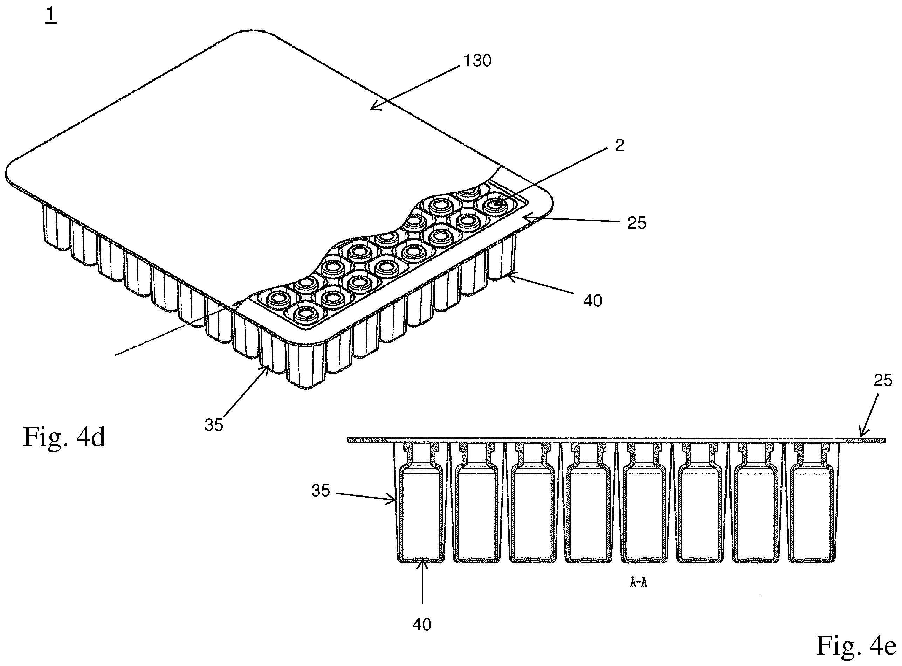

Gemäß der Fig. 2a ist ein als Haltestruktur wirkender flacher Träger 60 (im Stand der Technik häufig auch als sog. "Nest" bezeichnet) von sich rechtwinklig kreuzenden Querstegen 64 auf einer Grundplatte 61 ausgebildet. Auf diese Weise werden längliche, zylindrische Aufnahmen 65 zum Aufnehmen der Behälter 2 ausgebildet, wobei die Böden der Behälter 2 unmittelbar auf der Grundplatte 61 aufliegen. Die Grundplatte 61 kann aus einem sehr gut wärmeleitenden Material bestehen, insbesondere aus einem gut wärmeleitenden Metall oder auch aus einem Kunststoff, in den Metallpartikel eingebracht sind, um dessen Wärmeleitfähigkeit zu erhöhen. Der obere Teil der Fig. 2a stellt einen Transport- und Verpackungsbehälter 10 dar, der eine umlaufend ausgebildete Seitenwand 12, 14 und einen oberen Rand 15 aufweist und nachfolgend ausführlicher anhand der Fig. 4a beschrieben wird. Auf dem Boden des Transport- und Verpackungsbehälters 10 ist eine Struktur aus sich rechtwinklig kreuzenden Querstegen 55 ausgebildet bzw. angeordnet ist, die eine Mehrzahl von länglichen bzw. zylindrischen Hohlräumen 56 ausbilden, deren Öffnungsweite bevorzugt größer ist als die Öffnungsweite der zugeordneten länglichen Aufnahmen 65 auf der Grundplatte 61 bzw. die maximale Außenabmessung der Behälter 2 im jeweiligen Eingriffsbereichs. Die Querstege 55 auf dem Boden des Transport- und Verpackungsbehälters 10 sowie die Querstege 65 des flachen Trägers 60 sollen nur dazu dienen zu verhindern, dass die Seitenwände 4 der Behälter 2 einander unmittelbar berühren, beispielsweise aufgrund von Erschütterungen. Auf diese Weise können Beschädigungen der Seitenwände 4 der Behälter 2 weiter verhindert werden.

Gemäß den Figuren 2a und 2b sind die Seitenwände 63 des flachen Trägers 61 von einem Randsteg 62 eingefasst, der dann, wenn der Transport- und Verpackungsbehälter 10 auf die Grundplatte 61 aufgesetzt ist, auf dem oberen flanschartigen Rand 15 des Transport- und Verpackungsbehälters 10 aufliegt, wie in der Schnittdarstellung gemäß der Fig. 2b gezeigt. Wenn der Transport- und Verpackungsbehälter 10 auf die Grundplatte 61 aufgesetzt ist und der Rand 15 und der Randsteg 62 miteinander beispielsweise durch Verklebung oder Versiegelung hermetisch gegen die Umgebung abgedichtet sind, ist eine hermetisch abgedichtete Verpackungseinheit 1 ausgebildet, die durch Abziehen des Transport- und Verpackungsbehälters 10 von der Grundplatte 61 wieder geöffnet werden kann. Die halbkreisförmigen Öffnungen 66 im Rand 62 der Grundplatte können einer Positionierung oder zum besseren Greifen des flachen Trägers 60 oder einer Verkettung von benachbarten Trägern 60 dienen.

Die Behälter 2 können grundsätzlich lose in den Aufnahmen 65 des flachen Trägers 60 aufgenommen sein. Gemäß weiteren Ausführungsformen können die Behälter 2 auch reib- oder formschlüssig in den Aufnahmen 65 des flachen Trägers 60 aufgenommen sein. Gemäß der Fig. 2b sind auf dem Boden 11 des Transport- und Verpackungsbehälters 10 Abstandselemente 59 angeordnet, zwischen denen Öffnungen ausgebildet sind, über die beispielsweise ein Gas zur Sterilisierung über die Befüllöffnungen 7 in das Innenvolumen der Behälter 2 strömen kann. Üblicherweise würde der Transport- und Verpackungsbehälters 10 mit der Grundplatte 61 nach unten transportiert werden, sodass die Behälter 2 mit dem Kopf nach unten zeigend aufbewahrt und transportiert werden.

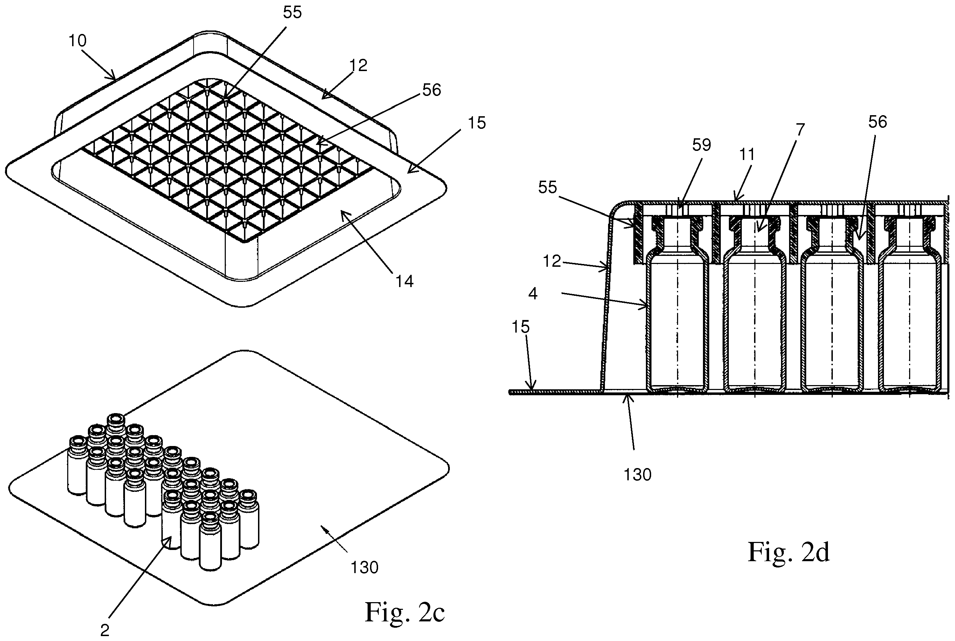

Bei einer weiteren Ausführungsform gemäß der Fig. 2c sind die Behälter 2 unmittelbar auf einer Schutz- oder Verpackungsfolie 130 angeordnet, auf die ein Transport- und Verpackungsbehälter 10 aufgesetzt wird (im Stand der Technik häufig auch als sog. "Tub" bezeichnet), wobei die auf dem Boden des Transport- und Verpackungsbehälters 10 angeordneten Querstege 55 eine unmittelbare Berührung der Seitenwände 4 der Behälter verhindern. An ihrem oberen Ende können die Behälter durch die Querstege 55 auch reibschlüssig fixiert, insbesondere geklemmt werden. Bei der Folie 130 kann es sich insbesondere um eine sterile, jedoch gasdurchlässige Folie handeln, insbesondere einem Kunststoff-Geflecht, wie beispielsweise Tyveck®. Alternativ kann der flache Träger 130 aus einem dünnen Metallblech oder einer dünnen Kunststoffplatte hergestellt sein. Bevorzugt werden Materialien mit einer möglichst hohen Wärmeleitfähigkeit, wie nachfolgend ausgeführt.

Bei dieser Ausführungsform können die in dem Transport- und Verpackungsbehälter 10 aufgenommenen Behälter 2 durch Einblasen eines Gases durch die Folie 130 hindurch sterilisiert werden. Damit einströmendes Gas in den Innenraum der Behälter 2 einströmen kann, sind zwischen dem Boden 11 des Transport- und Verpackungsbehälters 10 und dem oberen Rand der Behälter 2 Abstandshalter 59 vorgesehen, wie vorstehend anhand der Fig. 2b beschrieben, sodass die Behälter 2 nicht unmittelbar auf dem Boden 11 aufliegen.

Die Fig. 2d zeigt eine Draufsicht auf den auf dem Boden des Transport- und Verpackungsbehälters 10 ausgebildeten Einsatz, der auch herausnehmbar sein kann. Die Abstandhalter 59 können sich ausgehend von den Ecken einer jeweiligen Aufnahme 56 diagonal zur Mitte der jeweiligen Aufnahme 56 hin erstrecken. Die kreuzförmigen Abstandshaltestege 59 sind jedoch nicht miteinander verbunden, sodass im Bereich der Mitte einer jeweiligen Aufnahme 56 der obere Rand der Behälter ungehindert zugänglich ist. Die Fig. 2d zeigt eine Draufsicht auf den auf dem Boden des Transport- und Verpackungsbehälters 10 ausgebildeten Einsatz, der auch herausnehmbar sein kann.

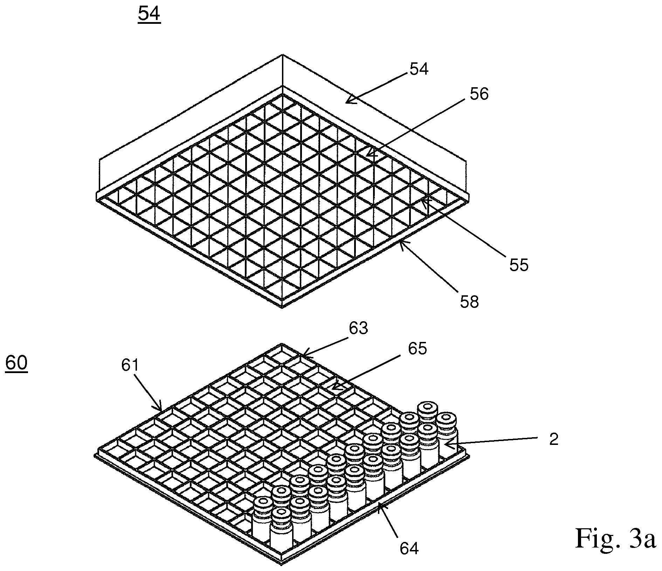

Die Fig. 3a zeigt eine weitere Variante eines flachen Trägers 60, bei der der vorgenannte Randabschnitt 62 der Grundplatte 61 (vgl. Fig. 2a) sehr schmal ausgebildet ist, so dass die Seitenwände 63 der Haltestruktur 60 unmittelbar in dem korrespondierend ausgebildeten Randabschnitt 58 des Einsatzes 54 aufgenommen sind, wie in der Schnittdarstellung gemäß der Fig. 3b dargestellt. Im zusammengesetzten Zustand gemäß der Fig. 3b ist ebenfalls eine Transport- und Verpackungseinheit 1 ausgebildet, die hermetisch gegen die Außenumgebung abgedichtet sein kann. Gemäß der Fig. 3b sind die Böden 3 der Behälter unmittelbar auf der Grundplatte 61 abgestützt. Nahe ihrem unteren Ende können die Behälter 2 in den von den Querstegen ausgebildeten länglichen Aufnahmen 65 grundsätzlich lose, d.h. mit einem gewissen radialen Spiel, aufgenommen sein oder in geeigneter Weise reib- oder formschlüssig fixiert sein.

Während vorstehend beschrieben wurde, dass der flache Träger den Transport- und Verpackungsbehälter verschließt oder versiegelt, werden nachfolgend anhand der Figuren 4a bis 4c weitere Ausführungsformen beschrieben, nach denen der flache Träger ein separates Element darstellt, das in den Transport- und Verpackungsbehälter nach der Behandlung oder Verarbeitung der Behälter gemeinsam mit diesen eingesetzt wird, bevor der Transport- und Verpackungsbehälter verschlossen oder versiegelt wird.

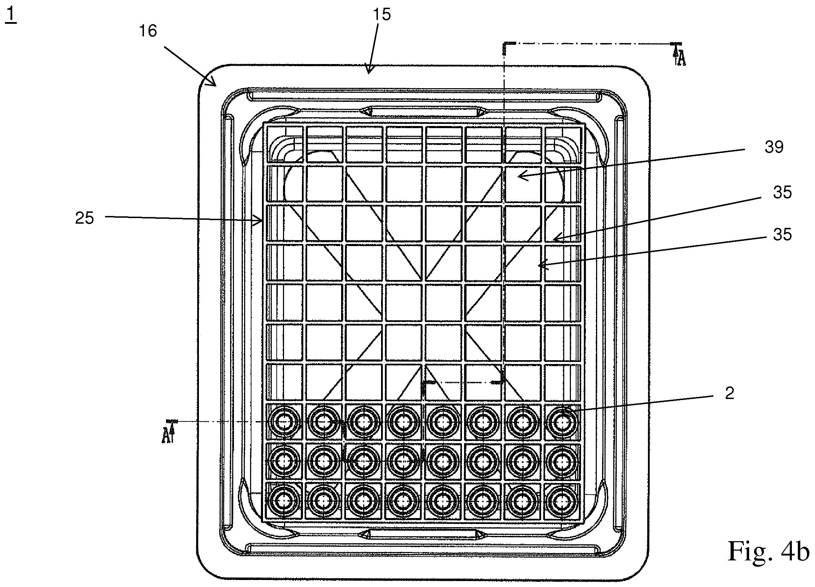

Bei dem dargestellten Ausführungsbeispiel gemäß der Fig. 4a ist der Träger 25 als separater Einsatz mit einer Mehrzahl von sich rechtwinklig kreuzenden Querstegen 35 ausgebildet, die eine Mehrzahl von länglichen bzw. zylindrischen Aufnahmen 39 mit quadratischem Querschnitt ausbilden, wobei die Aufnahmen 39 in einer Matrix-Anordnung angeordnet sind. Dabei werden die Behälter 2 entweder lose bzw. mit radialem Spiel in den Aufnahmen 39 aufgenommen oder an ihrem unteren Ende reib- oder formschlüssig von den Querstegen 35 oder in diesem Bereich vorgesehenen Haltemitteln fixiert, sodass die Böden der Behälter 2 unmittelbar auf dem Boden 40 des Trägers 25 aufliegen. Auf diese Weise wird auch nach dieser Ausführungsform ein guter thermischer Kontakt zwischen den Böden der Behälter und einer Kühlfläche gewährleistet. Grundsätzlich kann der Träger bzw. Einsatz 25 auch einstückig mit dem Boden 11 des Transport- und Verpackungsbehälters 1 ausgebildet sein. Die Fig. 4b zeigt den Transport- und Verpackungsbehälter 1 gemäß der Fig. 4a in einer schematischen Draufsicht.

Gemäß der Fig. 4a ist der Transport- und Verpackungsbehälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist. Die Ecken 16 des Behälters 10 sind zweckmäßig abgerundet ausgebildet. Die obere Seitenwand 14 kann unter einem geringen Neigungswinkel zur Senkrechten auf den Boden 11 geneigt ausgebildet sein, um das Einführen der Haltestruktur 25 zu erleichtern. Ein derartiger Behälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Behälter 10 aufgenommenen Haltestruktur 25 und der von dieser gehaltenen Behälter 2 zu ermöglichen.

Wenngleich in der Fig. 4a der Boden 11 des Transport- und Verpackungsbehälters 10 als geschlossen und einstückig mit der Seitenwand 12 ausgebildet dargestellt ist, kann das untere Ende des Transport- und Verpackungsbehälters 10 auch in der Art des oberen Endes geöffnet ausgebildet sein, insbesondere mit einem flanschartigen unteren Rand in der Art des oberen Rands 15 versehen sein, so dass die Böden der Behälter von der Unterseite des Transport- und Verpackungsbehälters 10 her frei zugänglich sind, beispielsweise für Verarbeitungsschritte in einem Steriltunnel oder in einem Gefriertrockenschrank.

Gemäß weiteren Ausführungsformen kann in dem Boden 11 oder der Seitenwand 12 zumindest eine Öffnung (nicht gezeigt) ausgebildet sein, durch die ein Gas zur Sterilisierung des Innenvolumens des Transport- und Verpackungsbehälters 10 einströmen kann. Eine solche Öffnung wird beispielsweise mit einer sterilen Kunststofffolie verschlossen, wie vorstehend anhand der Fig. 2c beschrieben. Bei der Schutzfolie kann es sich insbesondere um eine gasdurchlässige Kunststofffolie handeln, insbesondere um ein Geflecht aus Kunststofffasern, beispielsweise aus Polypropylen-Fasern (PP) oder auch um eine Tyvek®-Schutzfolie, die eine Sterilisation der in der Haltestruktur 25 aufgenommenen und verpackten Behälter 2 durch die Folie hindurch ermöglicht.

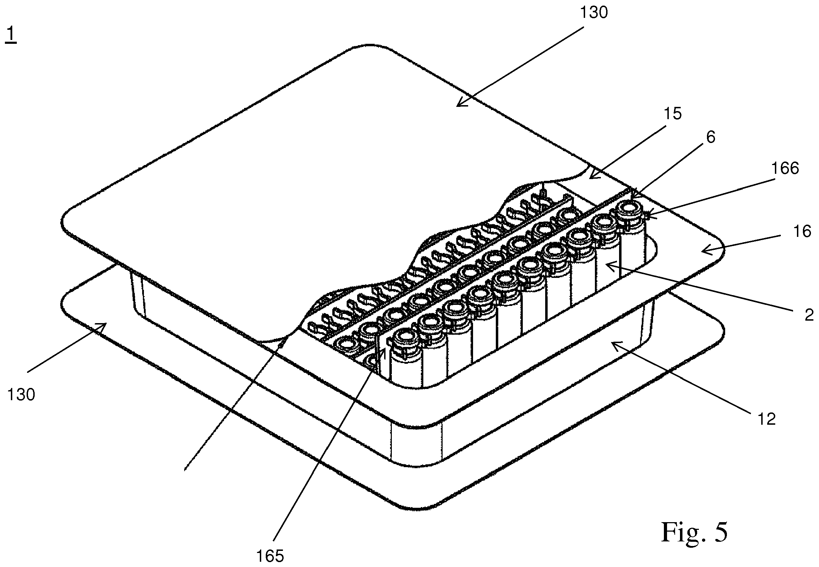

Nach der Variante gemäß der Fig. 5 sind beiden Enden des Transport- und Verpackungsbehälters offen ausgebildet und werden diese mittels einer sterilen Verpackungsfolie, wie vorstehend beschrieben, oder einer Platte verschlossen oder versiegelt. Gemäß der Fig. 5 ist der flache Träger als Kasten ausgebildet, mit umlaufenden Seitenwänden 12. Dieser Kasten wird von einer Mehrzahl von sich parallel erstreckenden und zueinander unter regelmäßigen Abständen zueinander beabstandeten Querstegen 165 in mehrere rechteckförmige Segmente unterteilt. Diese Querstege können in den Kasten auch eingehängt sein.

Auf den Oberflächen der Querstege 165 sind auf gleicher Höhe und unter regelmäßigen Abständen zueinander konkav gebogene Haltearme 166 angeordnet, die aus einem elastischen Kunststoff ausgebildet sind und entweder einstückig mit den Querstegen 165 ausgebildet sind oder an diesen befestigt oder angeformt sind. Die Haltearme 166 bilden Aufnahmen, in welche die Behälter 2 so von vorne her eingeführt werden können, dass deren Halsabschnitte formschlüssig umgriffen werden können und der obere Rand auf diesen abgestützt werden kann. In Längsrichtung der Behälter 2 betrachtet besteht ein Form- oder Reibschluss. Durch Aufbringen einer geeigneten axial wirkenden Kraft können die Behälter 2 jedoch in den von den Haltearmen 166 ausgebildeten Aufnahmen axial verschoben werden, beispielsweise in eine angehobene Stellung. Die Querstege 165 sind so in dem Kasten positioniert oder in diesen eingehängt, dass die Böden von sämtlichen Behältern 2 bündig mit dem unteren Rand des Kastens abschließen, sodass bei Ablage des Kastens auf einer Ablage- oder Kühlfläche eine unmittelbare Berührung der Böden von sämtlichen Behältern 2 mit der Ablage- oder Kühlfläche gewährleistet werden kann. Gemeinsam mit den auf dem oberen und unteren Rand aufgebrachten Kunststofffolien 130 wird so ebenfalls ein Transport- und Verpackungsbehälter 1 ausgebildet, in welchem die Mehrzahl von Behältern 2 steril und sicher verpackt sind und transportiert werden können.

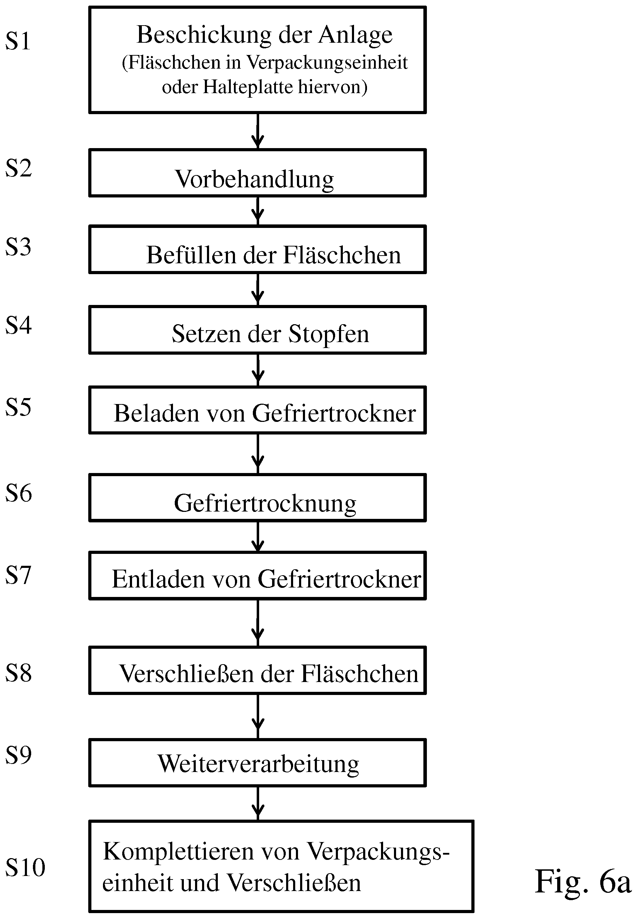

Die Fig. 6a zeigt ein schematisches Flussdiagramm eines Prozessschrittes zur Behandlung oder Verarbeitung von Behältern, nämlich beispielhaft ein Gefriertrocknungsprozess, wobei im Unterschied zu einem herkömmlichen Prozessschritt, wie vorstehend anhand der Fig. 1b beschrieben, eine Mehrzahl von Behältern in den Prozessschritten S1 bis S9, jedenfalls in den Prozessschritten S5 bis S7 gemeinsam an einem flachen Träger gehalten oder zumindest von diesem geführt aufgenommen sind, sodass die Böden der Behälter unmittelbar auf dem Boden des flachen Trägers aufliegen, wie vorstehend beschrieben.

Zunächst wird die Prozessanlage, beispielsweise ein Steriltunnel in dem Schritt S1 beschickt. Zu diesem Zweck werden die Fläschchen auf flachen Trägem, wie vorstehend beschrieben, angeordnet. Üblicherweise stehen die Fläschchen auf den Trägern aufrecht, d.h. mit der Befüllöffnung nach oben gerichtet. Bei weiteren Ausführungsformen, bei denen die Aufnahmen des Trägers so ausgelegt sind, dass die Fläschchen mit einer gewissen Haltekraft gehalten werden können, ist es jedoch grundsätzlich auch möglich, dass die Fläschchen vertikal nach unten gerichtet unterhalb des Trägers gehalten werden. In jedem Fall ist gewährleistet, dass die Böden der Fläschchen unmittelbar auf dem Boden des Trägers aufliegen. Solchermaßen beschickte Träger werden in dem Schritt S1 mittels einer Fördereinrichtung, beispielsweise einem Transportband, in die Prozessanlage transportiert. In dem Schritt S2 erfolgt optional eine Vorbehandlung, beispielsweise ein Auswaschen und eine Sterilisierung der Fläschchen. Anschließend werden die Fläschchen in dem Schritt S3 befüllt und optional in dem Schritt S4 geeignete Stopfen aufgesetzt, die noch offen sind.

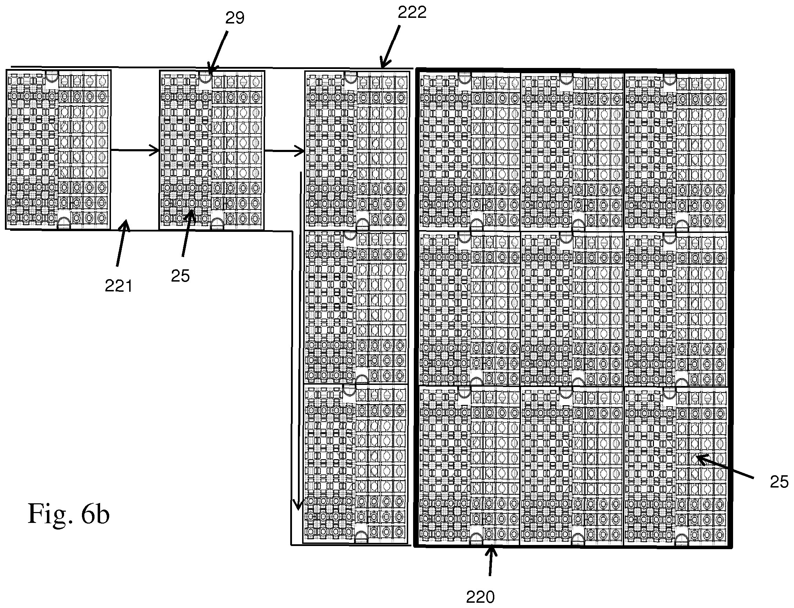

Anschließend wird der Gefriertrockner in dem Schritt S5 beladen. Dies ist schematisch in der Draufsicht gemäß der Fig. 6b dargestellt. Die flachen Träger 25 mit den von diesen in einer regelmäßigen zweidimensionalen Anordnung gehaltenen Behältern werden mittels einer Fördereinrichtung 221, beispielsweise eines Transportbands oder einer Rollenbahn, in Pfeilrichtung in Richtung zu einem Gefriertrockenschrank 220 gefördert. Dieser kann beispielsweise seitlich zu einer Haupt-Fördereinrichtung einer nicht dargestellten Prozessanlage angeordnet sein, von der die Träger 25 auf die Fördereinrichtung 221 umgesetzt bzw. umgelenkt und in Richtung zu dem Gefriertrockenschrank 220 gefördert werden. Vor dem Gefriertrockenschrank 220 befindet sich eine quer zur Fördereinrichtung 222 verlaufende Ablagefläche, auf der die Träger 25 gesammelt werden. Diese Sammlung der Träger 25 vor dem Gefriertrockenschrank 220 kann auch auf mehreren Ebenen erfolgen, in Entsprechung zu den Ebenen des Gefriertrockenschranks 220.

Die flachen Träger 25 mit den darauf angeordneten Behältern werden zur Gefriertrocknung in den Gefriertrockenschrank 220 eingebracht und liegen während der Gefriertrocknung unmittelbar auf einer Kühlfläche oder einem Kühlfinger des Gefriertrockenschranks 220 auf. Da die Böden der Behälter ihrerseits unmittelbar auf dem Boden des flachen Trägers 25 aufliegen, wird so ein guter thermischer Kontakt zu der Kühlfläche oder dem Kühlfinger des Gefriertrockenschranks 220 gewährleistet. Zu diesem Zweck wird es bevorzugt, wenn der flache Träger 25 aus einem gut wärmeleitenden Material besteht (beispielsweise aus einem Metall, Metallschaum, Metall-Verbundmaterial, Metall-Verbundmaterialschäum oder einem gut wärmeleitenden Kunststoff, in den insbesondere Metallpartikel eingebracht sind) und/oder mit einer möglichst geringen Materialstärke ausgebildet ist. Der flache Träger 25 kann auch aus einem faserverstärkten Kunststoff oder einem Kunststoff ausgebildet sein, dem zur Erhöhung seiner Wärmeleitfähigkeit Keramiken oder Metalle beigegeben sind. Bekanntermaßen haben faserverstärkte Kunststoffe eine höhere Wärmeleitfähigkeit bis 0,9 W/(K m) bei Kohlenstofffasern. Werden den Kunststoffen Keramiken oder Metalle beigegeben, so wird die Wärmeleitfähigkeit weiter vergrößert. Es entstehen die sogenannten wärmeleitenden Kunststoffe. So wird eine Wärmeleitfähigkeit von 20 W/(K m) erreicht.

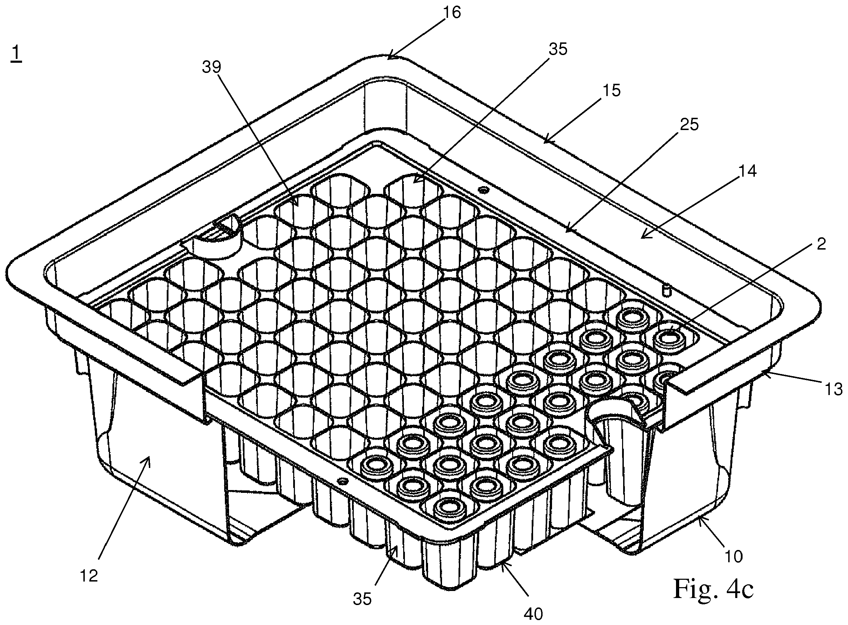

Nach dem Entladen des Gefriertrockenschranks 220 im Schritt S7 werden die Fläschchen verschlossen, insbesondere durch Crimpen oder Bördeln eines Metalldeckels auf dem oberen Rand der Fläschchen (Schritt S8). Nach einer optionalen Weiterverarbeitung (Schritt S9) werden die flachen Träger schließlich in einen Transport- und Verpackungsbehälter eingebracht (Ausführungsform nach der Fig. 4a oder Fig. 4c), der anschließend verschlossen oder versiegelt wird. Oder die Träger werden unmittelbar zum Verschliessen (Ausführungsform nach der Fig. 2a oder Fig. 3a) oder Versiegeln eines Transport- und Verpackungsbehälters (Ausführungsform nach der Fig. 2c) verwendet.

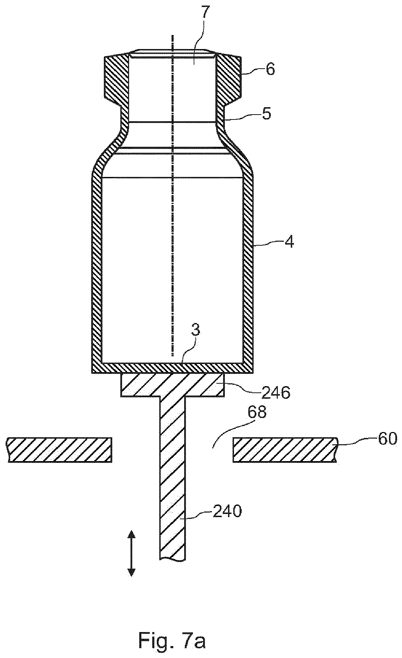

Damit der vorstehend beschriebene flache Träger auch für andere Prozessschritte verwendet werden kann, ohne dass die Fläschchen von dem Träger entnommen werden müssen, können weitere Merkmale vorgesehen sein, die nachfolgend anhand der Figuren 7a bis 7e beschrieben werden. So kann es erforderlich sein, dass die Fläschchen während eines Prozessschritts in eine angehobene Stellung angehoben werden müssen, in welcher diese nicht mehr unmittelbar auf dem Boden des flachen Trägers aufliegen. Zu diesem Zweck wird es bevorzugt, wenn eine Hubeinrichtung, beispielsweise eine vertikal verschiebbare Stange, von der Unterseite her auf die Fläschchen einwirken kann, weil dann das Eindringen von Verunreinigungen aufgrund von mechanischem Abrieb und dergleichen in das Innenvolumen der Fläschchen ausgeschlossen werden kann. Zu diesem Zweck können gemäß der Fig. 7a in dem Boden 60 eines nicht weiter dargestellten flachen Trägers Öffnungen 68 ausgebildet sein, beispielsweise in der Mitte einer jeweiligen Aufnahme 65 (vgl. Fig. 2a oder Fig. 3a). Die Öffhungsweise der Öffnung 68 ist einerseits so bemessen, dass der Boden 3 des Fläschchens nicht hindurch rutschen kann und somit der Boden 3 auf dem Boden 60 aufliegen kann, und andererseits so bemessen, dass die Hubeinrichtung, also bei dem Beispiel nach der Fig. 7a eine vertikal verschiebbare Stange 240 mit der an dem vorderen Ende vorgesehenen Auflagefläche 246 die Öffnung 68 durchgreifen kann. Insbesondere für Anwendungen bei der Gefriertrocknung wird es natürlich bevorzugt, dass die Öffnungsweite der Öffnung 68 möglichst gering ist, um einen möglichst guten thermischen Kontakt zu gewährleisten, wie vorstehend ausgeführt. Durch Anheben der Stange 240 kann das auf der Auflagefläche 246 aufliegende Fläschchen angehoben werden. Zum Ablegen des Fläschchens auf dem Boden 60 des flachen Trägers (nicht gezeigt) wird die Stange 240 mit der daran vorgesehenen Auflagefläche 246 durch die Öffnung 68 wieder vollständig nach unten abgezogen.

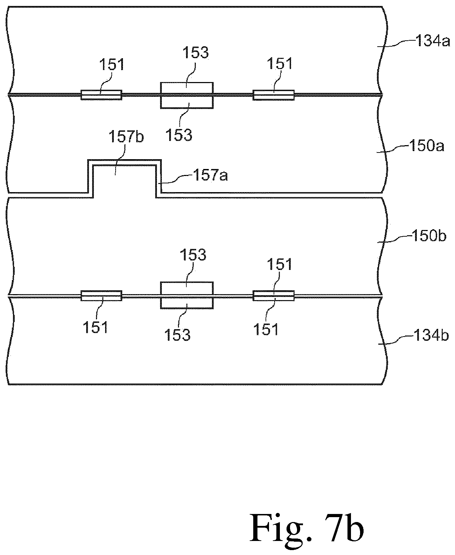

Die Fig. 7b zeigt in einem stark vergrößerten Teilschnitt und in Draufsicht eine weitere Variante eines flachen Trägers 134a, 134b (nur teilweise dargestellt), dessen Ränder 150a, 150b wegeklappt werden können, um die Grundfläche des jeweiligen Trägers weiter zu reduzieren, beispielsweise dann, wenn dieser mit den Behältern an eine beengte Weiterverarbeitungsstation übergeben werden soll, beispielsweise an einen Gefriertrockenschrank mit begrenzter Grundfläche. Zu diesem Zweck sind die Ränder 150a, 150b über Scharniere 151 mit dem jeweiligen Träger verbunden. Insbesondere können die Scharniere 151 als Filmscharniere oder Schnapp- bzw. Federscharniere aus einem Kunststoff einstückig mit dem Träger 134 ausgebildet sein.

Gemäß der Fig. 7b sind an den abnehmbaren oder wegschwenkbaren Elementen 150a, 150b Aussparungen 157a und/oder Vorsprünge 157b ausgebildet. Die Aussparungen 157a und/oder Vorsprünge 157b der abnehmbaren oder wegschwenkbaren Elemente 150 eines Trägers sind korrespondierend zu den Aussparungen 157a und/oder Vorsprüngen 157b der abnehmbaren oder wegschwenkbaren Elemente 150 eines unmittelbar benachbarten flächigen Trägers ausgebildet, sodass ein Formschluss zwischen diesen Aussparungen 157a und/oder Vorsprüngen 157b ausgebildet werden kann, um die gegenseitige Lage der Träger festzulegen und zu stabilisieren.

Auf der Oberseite der Träger 134a, 134b und der Ränder 150a, 150b sind an einander entsprechenden Positionen blockförmige Anschläge 153 vorgesehen, die im gegenseitigen Anschlag eine koplanare Ausrichtung der Ränder 150a, 150b und des Trägers 134 festlegen und ein Hochklappen der Ränder 150a, 150b verhindern. Die Träger können deshalb auch nur an den Rändern in einem Transport- und Verpackungsbehälter abgestützt werden.

Gemäß einer weiteren Ausführungsform (nicht dargestellt) können die Ränder 150 auch von dem Träger 134 abgenommen werden. Die Ränder 150 können selbstverständlich entlang von allen vier Längsseiten des Trägers 134 vorgesehen sein.

Die vorgenannten Vorsprünge 157b und Aussparungen 157a können auch unmittelbar am Rand eines flachen Trägers ausgebildet sein.

Durch die vorgenannte Auslegung der Vorsprünge 157b und Aussparungen 157a können zwei flache Träger jedoch grundsätzlich auch so miteinander verhakt werden, dass diese relativ zueinander in einer Längsrichtung und/oder in einer Querrichtung des Trägers unverschieblich sind und somit unmittelbar benachbarte Träger gemeinsam an einer Prozessstation vorbeigeführt werden oder diese durchlaufen können.

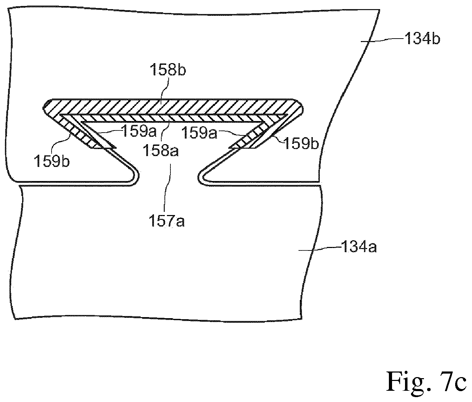

Ein Beispiel für die Auslegung der Vorsprünge 157b und Aussparungen 157a zur Erzielung einer Verhakung in der Art einer Schwalbenschwanzverbindung ist beispielhaft in der stark vergrößerten Teildraufsicht gemäß der Fig. 3c dargestellt. Die Vorsprünge 157b und Aussparungen 157a weisen, jeweils in Draufsicht betrachtet, eine insgesamt dreieckförmige oder polyedrische Grundfläche auf und sind korrespondierend zueinander ausgebildet, sodass diese unmittelbar miteinander verhakt werden können. Entlang den Rändern der Vorsprünge 157b und Aussparungen 157a sind zumindest abschnittsweise Seitenwände 158, 159 ausgebildet, die rechtwinklig von der Oberfläche des Trägers 134 abragen. Diese Seitenwände 158, 159 folgen der Kontur der zugeordneten Aussparung 157a bzw. des zugeordneten Vorsprungs 157b und wirken als Anschlag- und Führungsfläche, die ein Überreinandergleiten bzw. Aufschieben der Träger verhindern. Im verhakten Zustand gemäß der Fig. 7c liegen die Seitenwände 158a der unteren Halteplatte 134a unmittelbar an den Seitenwänden 158b der obere Halteplatte 134b an. Ferner liegen auch die abgewinkelten Seitenwände 159b der oberen Halteplatte 134b unmittelbar an den abgewinkelten Seitenwände 159a der unteren Halteplatte 134a an.

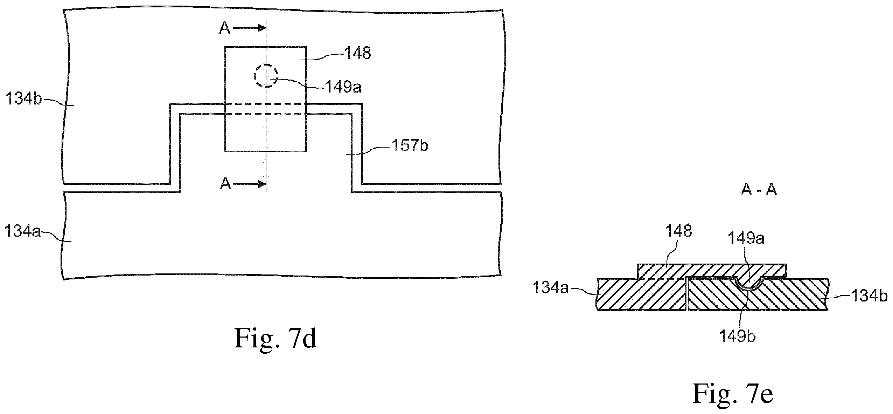

Die Fig. 7d zeigt als weiteres Beispiel für eine formschlüssige Verbindung in einer stark vergrößerten Teildraufsicht die Verbindung zweier Träger 134a, 134b nach einer weiteren Ausführungsform. Gemäß der Fig. 7d ragt von den hier rechteckförmig ausgebildeten Vorsprüngen 157b des unteren Trägers 134a eine elastische Zunge 148 in Richtung der zugeordneten Aussparung des oberen Trägers 134b rechtwinklig ab. Wie sich dem schematischen Teilschnitt gemäß der Fig. 7e entlang der Linie A-A gemäß der Fig. 7d entnehmen lässt, steht die elastische Zunge von der von den Trägern 134a, 134b aufgespannten Ebene vor, erstreckt sich jedoch parallel zu diesen. Am vorderen Ende der elastischen Zunge 148 ist ein kugelförmiger Vorsprung 149a ausgebildet, der in eine korrespondierend ausgebildete Aufnahme 149b auf der Oberseite des oberen Trägers 134b eingreift. Die Träger 134a, 134b können zur Verbindung aufeinander zu geschoben werden, bis das vordere Ende der elastischen Zunge 148 mit dem Vorsprung 149a schließlich in Anlage mit der Oberseite des oberen Trägers 134b gelangt. Bei weiterer Annäherung der beiden Träger 134a, 134b wird schließlich die elastische Zunge 148 nach oben gebogen, sodass der Vorsprung 149a entlang der Oberfläche des oberen Trägers 134b gleitet, bis dieser schließlich in den Bereich der Aufnahme 149b gelangt und aufgrund der Rückstellkraft der elastischen Zunge 148 in diese gedrückt wird. Die Elastizität der Zungen 148 und die Ausgestaltung der Formschlussgebilde 149a, 149b legen dabei in einfacher Weise die Stärke der lösbaren Verbindung zwischen den beiden Trägern 134a, 134b fest. Um ein Aufgleiten der beiden Träger 134a, 134b zu verhindern, können auch nach dieser Ausführungsform Anschlag- und Führungsflächen vorgesehen sein, insbesondere in Gestalt von Seitenwänden, die rechtwinklig von der Oberseite der Träger 134a, 134b abragen, wie vorstehend anhand der Fig. 7c beschrieben. Bei der Ausführungsform nach der Fig. 7d wären solche Seitenwände insbesondere seitlich neben den elastischen Zungen 148 vorzusehen.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Verfahren zur Behandlung oder Verarbeitung von Behältern (2), die zur Aufbewahrung von Substanzen für kosmetische, medizinische oder pharmazeutische Anwendungen dienen oder diese enthalten, insbesondere von Fläschchen (vials), wobei
die Behälter an einem Ende (7) offen ausgebildet sind,
die Behälter mittels einer Fördereinrichtung zur Behandlung oder Verarbeitung automatisiert an einer Prozessstation vorbeigeführt werden oder diese durchlaufen, und
die Behälter anschließend in einen kasten- oder wannenförmigen Transport- oder Verpackungsbehälter (1) eingebracht werden, der einen Boden (11), eine umlaufend ausgebildete Seitenwand (12, 14) zum Ausbilden eines Innenraums des Transport- oder Verpackungsbehälters und eine dem Boden des Transport- oder Verpackungsbehälters gegenüber liegende Einführöffnung aufweist, bei welchem Verfahren:
eine Mehrzahl von Behältern (2) in einer vorbestimmten Anordnung auf einem flachen Träger (62; 130) mit einem Boden (61; 130) angeordnet werden, sodass die Böden (3) der Behälter (2) unmittelbar auf dem Boden des flachen Trägers aufliegen;
die Behälter (2), während diese auf dem Boden des flachen Trägers aufliegen, von der Fördereinrichtung zu einer Prozessstation gefördert werden, um dort behandelt oder verarbeitet zu werden; und
der Transport- oder Verpackungsbehälter (1) nach der Behandlung oder Verarbeitung der Behälter so auf dem flachen Träger angeordnet wird, dass sämtliche Behälter der Mehrzahl von Behältern in dem Innenraum des kasten- oder wannenförmigen Transport- oder Verpackungsbehälters (1) aufgenommen sind.

2. Verfahren nach Anspruch 1, wobei auf dem Boden des flachen Trägers oder des Transport- oder Verpackungsbehälters Positionierungsmittel (35; 64) ausgebildet sind, die mit den Behältern so zusammenwirken, um die vorbestimmte Anordnung der Mehrzahl von Behältern (2) festzulegen.

3. Verfahren nach Anspruch 2, wobei
die Positionierungsmittel (35; 64) eine Kollision von unmittelbar benachbarten Behältern (2) verhindern, während diese auf dem Boden des flachen Trägers (62; 130) aufliegen oder in dem Transport- oder Verpackungsbehälter (1) aufgenommen sind, und/oder
wobei die Positionierungsmittel als zylindrische oder polyedrische Aufnahmen (39; 65) ausgebildet sind, und die Behälter (2) zumindest abschnittsweise in die Aufnahmen eingeführt werden.

4. Verfahren nach einem der Ansprüche 2 oder 3, wobei zwischen den Positionierungsmitteln Öffnungen ausgebildet sind, die von einer Hebeeinrichtung (240) durchgriffen werden, wobei die Hebeeinrichtung die Behälter (2) zur Behandlung oder Verarbeitung in oder an der Prozessstation in eine angehobene Position anhebt, in welcher die Böden (3) der Behälter (2) nicht unmittelbar auf dem flachen Träger aufliegen.

5. Verfahren nach Anspruch 4, wobei die Behälter (2) in der angehobenen Position weiterhin mit den Positionierungsmitteln (35; 64) zusammenwirken, sodass die vorbestimmte Anordnung in der angehobenen Position weiterhin von den Positionierungsmitteln festgelegt wird.

6. Verfahren nach einem der vorhergehenden Ansprüche, wobei die Behälter (2) so in dem Transport- oder Verpackungsbehälter (1) aufgenommen werden, dass das offene Ende (7) dem Boden (11) des Transport- oder Verpackungsbehälters (1) zugewandt ist.

7. Verfahren nach einem der vorhergehenden Ansprüche, wobei in dem flachen Träger oder dem Transport- oder Verpackungsbehälter (1) zumindest eine Öffnung ausgebildet ist, durch welche ein Gas zum Sterilisieren des Innenraums des Transport- oder Verpackungsbehälters (1) einströmt.

8. Verfahren nach einem der vorhergehenden Ansprüche, wobei der flache Träger (62; 130) aus einem Metall, einem metallhaltigen Material oder einem Kunststoff mit einer hohen Wärmeleitfähigkeit besteht, insbesondere aus einem Kunststoff mit in diesen eingebrachten Metallpartikeln, und
wobei die Prozessstation ein Gefriertrockenschrank ist, in welchem ein Gefriertrocknungsprozess ausgeführt wird, wobei die Böden (3) der Behälter (2) während des Gefriertrocknungsprozesses unmittelbar auf dem flachen Träger aufliegen und der flache Träger unmittelbar auf einer Kühlfläche des Gefriertrockenschranks aufliegt.

9. Verfahren nach einem der vorhergehenden Ansprüche, wobei der Transport- oder Verpackungsbehälter mittels einer gasdurchlässigen Kunststofffolie (130) verschlossen oder versiegelt wird, insbesondere mittels einer Kunststofffolie, die aus einem gasdurchlässigen Geflecht von Kunststofffasern ausgebildet ist und insbesondere eine Tyveck®-Folie ist.

10. Verfahren nach Anspruch 9, wobei die gasdurchlässige Kunststofffolie als der flache Träger (130) verwendet wird.

11. Verfahren nach einem der vorhergehenden Ansprüche, wobei jeweils benachbarte Träger zur Behandlung oder Verarbeitung so miteinander unmittelbar verbunden werden, dass diese relativ zueinander in einer Längsrichtung und/oder in einer Querrichtung des Trägers unverschieblich sind und die unmittelbar benachbarten Träger gemeinsam an der Prozessstation vorbeigeführt werden oder diese durchlaufen.

12. Verfahren nach einem der vorhergehenden Ansprüche, wobei Ränder (150) des flachen Trägers als abnehmbare oder wegschwenkbare Elemente (150) ausgebildet sind und zur Behandlung oder Verarbeitung abgenommen oder weggeschwenkt werden, um die Grundfläche des Trägers (130) zu reduzieren.

13. Verfahren nach Anspruch 12, wobei an den abnehmbaren oder wegschwenkbaren Elementen (150) und/oder an Rändern von unmittelbar benachbarten Trägern zueinander korrespondierend ausgebildete Formschlussgebilde ausgebildet sind, die miteinander zur Behandlung oder Verarbeitung der Behälter in einen formschlüssigen Eingriff überführt werden.

14. Verfahren nach Anspruch 13, wobei
die Formschlussgebilde als Aussparungen und korrespondierend dazu ausgebildete Vorsprünge ausgebildet sind, wobei die Aussparungen (157a) und/oder Vorsprünge (157b) an dem Träger korrespondierend zu den Aussparungen (157a) und/oder Vorsprüngen (157b) eines unmittelbar benachbarten Trägers ausgebildet sind, wobei Grundflächen der Aussparungen und/oder Vorsprünge, jeweils in Draufsicht betrachtet, insbesondere verschieden zu einer Rechteckform sind und unmittelbar korrespondierend zueinander ausgebildet sind, oder
wobei die Formschlussgebilde an einem ersten der beiden benachbarten Träger eine elastische Zunge (148) mit einem daran darauf ausgebildeten Rastvorsprung (149a) oder einer darauf ausgebildeten Rastaussparung sowie an dem zweiten der beiden benachbarten Träger eine korrespondierend zu dem Rastvorsprung (149a) ausgebildete Aufnahme (149b) oder einen korrespondierend zu der Rastaussparung ausgebildeten Vorsprung aufweisen, die miteinander in den formschlüssigen Eingriff überführt werden.

15. Verwendung eines flachen Trägers (62; 130), der einen Boden (61; 130) aufweist und
der zum Verschließen oder Versiegeln eines kasten- oder wannenförmigen Transport- oder Verpackungsbehälters dient, der einen Boden (11), eine umlaufend ausgebildete Seitenwand (12, 14) zum Ausbilden eines Innenraums des Transport- oder Verpackungsbehälters und eine dem Boden des Transport- oder Verpackungsbehälters gegenüber liegende Einführöffnung aufweist, oder
der in einem verschlossenen oder versiegelten Transport- oder Verpackungsbehälter zum Transport aufgenommen ist,
als Träger für eine Mehrzahl von Behältern (2), die zur Aufbewahrung von Substanzen für kosmetische, medizinische oder pharmazeutische Anwendungen dienen oder diese enthalten, insbesondere von Fläschchen (vials), bei der Behandlung oder Verarbeitung der Behälter an oder in einer Prozessstation, wobei die Mehrzahl von Behältern (2) in einer vorbestimmten Anordnung so an dem flachen Träger angeordnet sind, dass die Böden (3) der Behälter (2) unmittelbar auf Boden des flachen Trägers aufliegen, und wobei sämtliche Behälter der Mehrzahl von Behältern in dem Innenraum des kasten- oder wannenförmigen Transport- oder Verpackungsbehälters (1) aufgenommen sind.

## Claims

1. A process for treatment or processing of containers (2) used for storing substances for cosmetic, medical or pharmaceutical applications, or containing those, in particular of vials, wherein
the containers are formed to be open at one end (7),
the containers are conveyed past a processing station by a conveying device for automated treatment or processing or are conveyed through it, and
the containers are then inserted into a box-shaped or tub-shaped transport or packaging container (1) having a bottom (11), a circumferential side-wall (12, 14) for forming an interior space of the transport or packaging container and an insertion opening opposite to the bottom, in which process:
a plurality of containers (2) are placed on a flat carrier having a base (62; 130), so that the bottoms (3) of the containers (2) rest directly on the base of the flat carrier;
the containers (2), while resting on the base of the flat carrier, are conveyed to a processing station by said conveying device in order to be treated or processed there; and
the transport or packaging container (1) is arranged on the flat carrier after the treatment or processing of the containers such that all containers of the plurality of containers are accommodated in the interior space of the box-shaped or tub-shaped transport or packaging container (1).

2. The process of claim 1, wherein positioning means (35; 64) are formed on the base of the flat carrier or on the bottom of the transport or packaging container, which cooperate with the containers, so as to define the predetermined arrangement of the plurality of containers (2).

3. The process of claim 2, wherein the positioning means (35; 64) prevent a collision of directly adjacent containers (2) while these are resting on the base of the flat carrier (62; 130) or are accommodated in the transport or packaging container (1); and/or
wherein the positioning means are formed as cylindrical or polygonal receptacles (39; 65) and the containers (2) are at least partially inserted into the receptacles.

4. The process of claim 2 or 3, wherein openings are formed between the positioning means through which a lifting device (240) extends to, wherein the lifting device raises the containers (2) to a raised position for treatment or processing in or at the processing station, in which the bottoms (3) of the containers (2) do not directly rest on the flat carrier.

5. The process of claim 4, wherein the containers (2) continue cooperating with the positioning means (35; 64) in the raised position, so that the predetermined arrangement is still defined by the positioning means in the raised position.

6. The process of any of the preceding claims, wherein the containers (2) are accommodated in the transport or packaging container (1) such that the open ends (7) face the bottom (11) of the transport or packaging container (1).

7. The process of any of the preceding claims, wherein at least one opening is formed in the flat carrier or in the transport or packaging container (1) through which a gas flows for sterilization of the interior space of the transport or packaging container (1).

8. The process of any of the preceding claims, wherein the flat carrier (62; 130) is made of a metal, a metal-containing material or a plastic material having a high thermal conductivity, in particular of a plastic material having metal particles embedded therein, and
wherein the processing station is a freeze-dryer, in which a lyophilization process is performed, wherein the bottoms (3) of the containers (2) rest directly on the flat carrier during the lyophilization process, and the flat carrier rests directly on a cooling surface of the freeze-dryer.

9. The process of any of the preceding claims, wherein the transport or packaging container is closed or sealed by means of a gas permeable plastic foil (130) formed of a gas permeable fabric of synthetic fibers and which is in particular a Tyveck®-foil.

10. The process of claim 9, wherein the gas-permeable plastic foil is used as the flat carrier (130).

11. The process of any of the preceding claims, wherein respectively adjacent carriers are directly connected to one another for treatment or processing such that these cannot be moved relative to each other in a longitudinal direction and/or in a transverse direction of the carrier and the directly adjacent carriers are conveyed past the process station or through it together.

12. The process of any of the preceding claims, wherein rims (150) of the flat carrier are formed as removable members (150) or as members that can be pivoted away and are removed or pivoted away for treatment or processing in order to reduce the base area of the carrier (130).

13. The process of claim 12, wherein positive-fit structures, which are formed correspondingly to each other, are formed on the removable or pivotal members (150) and/or at rims of directly adjacent carriers, which are brought into a positive-fit engagement with each other for the treatment or processing of the containers.

14. The process of claim 13, wherein
the positive-fit structures are formed as recesses and correspondingly formed projections, wherein the recesses (157a) and/or projections (157b) on the carrier are formed correspondingly to the recesses (157a) and/or projections (157b) of a directly adjacent carrier, wherein the base areas of the recesses and/or projections, each if viewed in a plan view, are in particularly different to a rectangular shape and are formed such that they directly correspond to each other, or
wherein the positive-fit structures comprise on a first of the two adjacent carriers a resilient tongue (148) with a locking projection (149a) formed thereon or with a locking recess formed thereon and on the second of the two adjacent carriers a receptacle (149b) formed correspondingly to the locking projection or correspondingly to the locking recess, which are brought into a positive-fit engagement with each other.

15. A use of a flat carrier (62; 130) having a base (61; 130) and which serves to close or seal a box-shaped or tub-shaped transport or packaging container which has a bottom (11), a circumferential side-wall (12, 14) for forming an interior space of the transport or packaging container and an insertion opening opposite to the bottom of the transport or packaging container, or
which is accommodated in a closed or sealed transport or packaging container for transport thereof,
as a carrier for a plurality of containers (2) used for storing substances for cosmetic, medical or pharmaceutical applications, or containing those, in particular vials, during the treatment or processing of the containers at or in a processing station, wherein the plurality of containers (2) are arranged in a predetermined arrangement on the flat carrier such that the bottoms (3) of the containers (2) rest directly on the base of the flat carrier, and wherein all the containers of the plurality of containers are accommodated in the interior space of the box-shaped or tub-shaped transport or packaging container (1).

## Revendications

1. Un procédé de traitement ou de traitement de récipients (2) utilisé pour le stockage de substances pour des applications cosmétiques, médicales ou pharmaceutiques, en particulier des flacons, dans lequel
les récipients sont formé pour être ouverts à une extrémité (7),
les récipients sont transportés par un convoyeur vers une station de traitement pour une transformation ou traitement automatisé, ou pour y passer, et
les récipients sont ensuite introduits dans un conteneur de transport ou de conditionnement (1), en forme de boîte ou de forme tubulaire, ayant un fond (11), une paroi latérale circonférentielle (12, 14) pour former un espace intérieur pour le transport ou le conditionnement de récipients et une ouverture d'insertion opposée au fond, dans lequel le procédé :
une pluralité de récipients (2) sont placés sur un support plat ayant une base (62, 130), de telle façon que les fonds (3) des récipients (2) reposent directement sur la base du support plat ;
les récipients (2), tout en reposant sur le support plat, sont acheminés par ledit convoyeur vers une station de traitement afin d'y être traité ou transformé ; et
le conteneur de transport ou de conditionnement (1) est agencé sur le support plat après le traitement ou la transformation des récipients de sorte que tous les récipients au sein de la pluralité de récipients soient logés dans le conteneur de transport ou de conditionnement (1).

2. Le procédé de la revendication 1, dans lequel des moyens de positionnement (35; 64) sont formés sur le fond du support plat ou sur le fond du conteneur de transport ou de conditionnement, qui coopèrent avec les récipients pour définir l'agencement prédéterminé de la pluralité de récipients (2).

3. Le procédé de la revendication 2, dans lequel les moyens de positionnement (35; 64) empêchent une collision de récipients (2) immédiatement adjacents tant qu'ils reposent sur le support plat (62; 130) ou tant qu'il sont logées dans le conteneur de transport ou de conditionnement (1), et/ou
dans lequel les moyens de positionnement sont formés sous forme de récipients cylindriques ou polyédriques (39; 65), et les récipients (2) sont introduits au moins par sections dans les réceptacles.

4. Le procédé de la revendication 2 ou 3, dans lequel des ouvertures sont formées entre les moyens de positionnement, au travers desquels pénètre un dispositif de levage (240), dans lequel le dispositif de levage soulève les récipients (2) vers une position surélevée pour un traitement ou une transformation dans ou au niveau de la station de traitement, dans lequel les fonds (3) des récipients (2) ne reposent pas directement sur le support plat.

5. Le procédé de la revendication 4, dans lequel les récipients (2) continuent de coopérer avec les moyens de positionnement (35, 64) dans la position surélevée, de telle sorte que l'agencement prédéterminé dans la position surélevée continue à être défini par les moyens de positionnement dans la position surélevée.

6. Le procédé de l'une quelconque des revendications précédentes, dans lequel les récipients (2) sont logés dans le conteneur de transport ou de conditionnement (1) de telle sorte que l'extrémité ouverte (7) fait face au fond (11) du conteneur de transport ou de conditionnement (1).

7. Le procédé de l'une quelconque des revendications précédentes, dans lequel au moins une ouverture est formée dans le support plat ou dans le conteneur de transport ou de conditionnement (1) au travers de laquelle un gaz de stérilisation s'écoule à l'intérieur du conteneur de transport ou de conditionnement (1).

8. Le procédé de l'une quelconque des revendications précédentes, dans lequel le support plat (62 ; 130) consiste en un métal, un matériau contenant du métal ou une matière plastique à haute conductivité thermique, en particulier une matière plastique dans laquelle sont incorporées des particules métalliques, et
dans lequel la station de traitement est une armoire de lyophilisation dans laquelle est effectué un procédé de lyophilisation, dans laquelle les fonds (3) des récipients (2) reposent directement sur le support plat pendant le processus de lyophilisation et le support plat repose directement sur une surface de refroidissement du lyophilisateur.

9. Le procédé de l'une quelconque des revendications précédentes, dans lequel le conteneur de transport ou de conditionnement est fermé ou scellé au moyen d'un film plastique perméable aux gaz (130) formé à partir d'un matériau de fibres synthétiques perméable aux gaz et en particulier d'un film Tyveck®.

10. Le procédé de la revendication 9, dans lequel le film plastique perméable aux gaz est utilisé en tant que support plat (130).

11. Le procédé de l'une quelconque des revendications précédentes, dans lequel les supports adjacent sont respectivement et directement reliés les uns aux autres pour une transformation ou un traitement de telle sorte qu'ils sont immobiles les uns par rapport aux autres dans une direction longitudinale et/ou dans une direction transversale du support et les supports immédiatement adjacents sont transportés ensemble à la station de traitement, ou y passent ensemble.

12. Le procédé de l'une quelconque des revendications précédentes, dans lequel les bords (150) du support plat sont formés en tant qu'éléments amovibles (150) ou en tant qu'éléments pivotants (150) et sont enlevés ou basculés pour la transformation ou le traitement afin de réduire l'encombrement du support (130).

13. Le procédé de la revendication 12, dans lequel des structures à ajustement de forme, qui sont formées en correspondantes les unes aux autres, sont formées sur les éléments amovibles ou pivotants (150) et/ou au niveau des bords de supports immédiatement adjacents, qui sont apportés dans un engagement d'ajustement de forme les uns aux autres pour la transformation ou le traitement des récipients.

14. Le procédé de la revendication 13, dans lequel
les structures à ajustement de forme sont formées sous la forme d'évidements et de saillies correspondantes, dans lequel les évidements (157a) et/ou les saillies (157b) sur le support sont formées en correspondance avec les évidements (157a) et/ou les saillies (157b) d'un support directement adjacent, dans lequel les surfaces de base des évidements et/ou saillies, chacune considérée dans une vue plane, sont en particulier différentes d'une forme rectangulaire et sont formées de sorte qu'elles correspondent l'une à l'autre, ou
dans lequel les structures à ajustement de forme comporte sur une première parmi les deux supports adjacents une languette élastique (148) une saillie de verrouillage (149a) formée sur celle-ci ou un évidement de verrouillage formé sur celle-ci, et comporte sur une seconde parmi les deux supports adjacents un réceptacle (149b) formé en correspondance avec la saillie de verrouillage (149a) ou en correspondance avec l'évidement de verrouillage, qui sont amené dans un engagement d'ajustement de forme l'une vis-à-vis de l'autre.

15. Une utilisation d'un support plat (62; 130) ayant une base (61 ; 130) et qui sert à la fermeture ou au scellement d'un récipient de transport ou de conditionnement en forme de boîte ou de forme tubulaire ayant un fond (11), une paroi latérale circonférentielle (12, 14) pour former un espace intérieur du récipient de transport ou de conditionnement, et une ouverture d'insertion opposée au fond du récipient de transport ou de conditionnement, ou
qui est logé dans un conteneur de transport et de conditionnement fermé ou scellé pour son transport ;
comme un support pour une pluralité de récipients (2) utilisé pour le stockage de substances cosmétiques, médicales ou pharmaceutiques, ou pour en contenir, en particulier des flacons, durant la transformation ou le traitement des récipients dans ou au niveau d'une station de traitement, dans laquelle la pluralité de récipients (2) sont disposées dans un agencement prédéterminé sur le support plat de telle sorte que les fonds (3) des récipients (2) reposent directement sur la base du support plat, et dans laquelle tous les récipients de la pluralité de récipients sont logés dans l'espace intérieure du conteneur de transport et de conditionnement (1) en forme de boîte ou de forme tubulaire.
